# EUROPÄISCHE PATENTANMELDUNG

(11) **EP 1 250 900 A2**
(43) Veröffentlichungstag der Anmeldung: **23.10.2002**
(21) Anmeldenummer: 02003945.9
(22) Anmeldetag: 22.02.2002
(51) Int. Cl.: A61F 9/011

(54) **Vorrichtung zur Glaukombehandlung**

(30) Priorität: 18.04.2001 DE 10118933
(71) Anmelder: Glautec AG, 90425 Nürnberg (DE)
(72) Erfinder: Neuhann,Thomas, Prof., 80637 München (DE)
(74) Vertreter: Matschkur, Lindner Blaumeier Patent- und Rechtsanwälte

(57) **Zusammenfassung**

Vorrichtung zur Glaukombehandlung mit einem Laserkatheder und einer lichtführenden Faseranordnung, an deren proximalem Ende Licht einkoppelbar ist und an deren distalem Ende eine Lichtaustrittsfläche vorgesehen ist, mit einer drucksensitiven Vorrichtung zur Erzielung eines vorgegebenen Anlagedrucks der Lichtaustrittsfläche.

## Beschreibung

Die Erfindung bezieht sich auf eine Vorrichtung zur Glaukombehandlung mit einem Laserkatheder und einer lichtführenden Faseranordnung, an deren Ende Licht einkoppelbar ist und deren distalem Ende eine Lichtaustrittsfläche vorgesehen ist.

Zur Glaukombehandlung, also zur Beseitigung eines Überdrucks im Augapfel, gibt es neben medikamentösen Verfahren eine Reihe von chirurgischen Verfahren. Unter anderem ist dabei auch bereits eine Vorrichtung der vorstehend beschriebenen Art vorgeschlagen worden, bei welcher mittels UV-Licht das vorzugsweise über einen Excimerlaser erzeugt und über Lichtleiter in das Augeninnere geführt wird, das schwammartige Trabekelwerk, durch das Kammerwasser aus der vorderen und hinteren Augenkammer fließt, lokal abgetragen wird, so dass das Kammerwasser leichter in den Schlemm'schen Kanal gelangen kann, durch den es schließlich abgeführt wird.

Zum Einsatz dieser Vorrichtung ist es erforderlich, das Auge lokal zu öffnen, um mit Hilfe des lichtleitenden Laserkatheters Licht in unmittelbare Nähe des zu perforierenden Gewebes des Trabekelwerkes zu leiten. Notwendig ist es dabei, die Lichtaustrittsfläche genau vor dem Schlemm'schen Kanal zu positionieren, um genau an dieser Stelle das Trabekelwerk zu durchlöchern und den Abfluss in den Schlemm'schen Kanal sicherzustellen. Diese Positionierung des distalen Endes des Laserkatheters ist aber sehr schwierig und macht dadurch den Einsatz einer solchen Glaukombehandlungsvorrichtung bis zu einem gewissen Grad zu einem Glücksspiel, bei dem es darauf ankommt, ob man die richtige Position beim Einführen des Laserkatheters wirklich gefunden hat oder nicht.

Ein weiteres wesentliches Problem beim Einsatz einer derartigen Vorrichtung zur Glaukombehandlung mit einem Laserkatheder besteht in der Aufrechterhaltung eines vorgegebenen Anpressdrucks, damit nicht das Trabekelwerk mechanisch von der lichtführenden Faseranordnung durchstochen wird und diese lichtführende Faseranordnung auch nicht von der gewünschten Anlagestelle seitlich wegrutschen kann.

Der Erfindung liegt daher die Aufgabe zugrunde eine Vorrichtung der eingangs genanten Art so auszugestalten, dass die Positionierung des distalen Endes der lichtführenden Faseranordnung unter Sicherstellung eines vorgegebenen Anpressdrucks gewährleistet ist.

Zur Lösung dieser Aufgabe ist eine Vorrichtung zur Glaukombehandlung der eingangs genannten Art gekennzeichnet durch eine drucksensitive Vorrichtung zur Erzielung eines vorgegebenen Anlagedrucks der Lichtaustrittsfläche.

Eine derartige drucksensitive Vorrichtung kann ein auf das distale Ende aufsteckbares elastisches Bauteil mit einer abwinkelbaren Andrücklippe sein, wobei diese Andrücklippe ggf. auch noch so ausgestaltet sein kann, dass sie im Kammerwinkel abstützend wirkt und damit ein Abgleiten der Faseranordnung an der zuvor platzierten Stelle verhindert.

Bei einer derartigen Andrückeinrichtung mit einer abwinkelbaren Andrücklippe ergibt sich nicht nur durch die Kraft, die für das Abwinkeln erforderlich ist, eine Reaktionskraft für den Operateur, die das Anliegen der Spitze signalisiert, sondern er kann auch das Abwinkeln der Andrücklippe im Mikroskop erkennen und somit die richtige Position durch Beobachtung der sich abwinkelnden Andrücklippe ermitteln.

In Weiterbildung der Erfindung kann vorgesehen sein, dass der Laserkatheder im Bereich außerhalb des Auges zwei axial gegen eine Federanordnung gegeneinander verschiebbare Abschnitte aufweist, wobei bevorzugt eine optische und/oder akustische Anzeigevorrichtung zur Signalisierung einer Erreichung oder Überschreitung des vorgegebenen Anpressdrucks vorgesehen sein kann.

In weiterer Ausgestaltung der Erfindung kann auch eine Überlastsicherung vorgesehen sein, die bei Überschreitung eines vorgebbaren Anpressdrucks der Lichtaustrittsfläche der lichtführenden Faseranordnung die kraftschlüssige Verbindung der Abschnitte unterbricht, sodass ein versehentliches Durchstechen sicher verhindert ist.

Die drucksensitive Vorrichtung kann auch mit nichtfedernden Bauteilen ausgestaltet sein (z. B. Membran-/ Kristalltechnik); unter der Vorraussetzung, dass eine Wahrnehmung des erreichten Druckes durch den Anwender gewährleistet ist, wobei bevorzugt eine optische und/oder akustische Anzeigevorrichtung zur Signalisierung einer Erreichung oder Überschreitung des vorgegebenen Anpressdrucks vorgesehen sein kann.

Schließlich liegt es auch noch im Rahmen der Erfindung, dass das distale Ende der lichtführenden Faseranordnung mit einer ein Abgleiten der angedrückten Lichtaustrittsfläche verhindernden, vorzugsweise aufsteckbaren Abgleitsicherung versehen ist, wobei diese Abgleitsicherung einen Dorn aufweisen kann, oder aber auch dadurch gebildet sein kann, dass das Aufsteckglied aus einem Antirutschmaterial besteht, das am Trabekelwerk haftet.

Weitere Vorteile, Merkmale und Einzelheiten der Erfindung ergeben sich aus der nachfolgenden Beschreibung eines Ausführungsbeispiels sowie anhand der Zeichnung. Dabei zeigen:
- Fig. 1: Eine schematische Darstellung einer erfindungsgemäßen Vorrichtung mit einer abwinkelbaren Andrücklippe,
- Fig. 2: eine vergrößerte Ausschnittsdarstellung des Bereichs II in Fig. 1,
- Fig. 3: eine schematische Darstellung einer zweiten Ausführungsform einer erfindungsgemäßen Vorrichtung mit federnd axial gegeneinander verschiebbaren Abschnitten und
- Fig. 4: eine vergrößerte Ausschnittsdarstellung des distalen Endes der lichtleitenden Faseranordnung einer weiteren Ausführungsform einer erfindungsgemäßen Glaukombehandlungsvorrichtung mit einer Abgleitsicherung in Form eines Dorns.

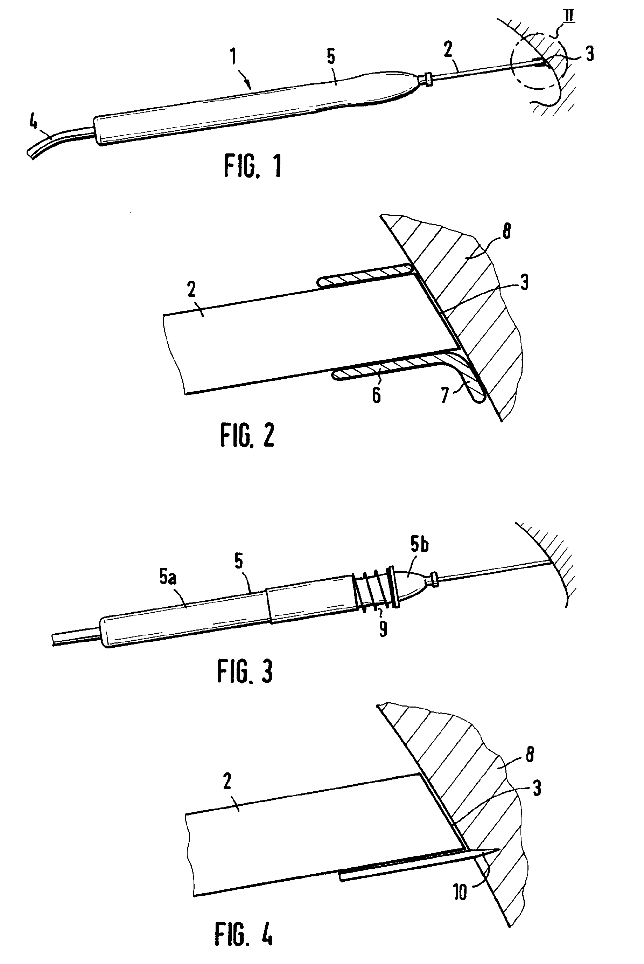

Die in Fig. 1 schematisch gezeigte Glaukombehandlungsvorrichtung umfasst einen Laserkatheder 1 mit einer lichtführenden Faseranordnung 2 an deren distalem Ende eine Lichtaustrittsfläche 3 vorgesehen ist. Die Zuführung des Lichts von einer nicht dargestellten Laserquelle erfolgt über einen Lichtleiter 4. Gezeigt ist dabei auch schematisch der Handgriff 5 des Laserkatheders mit Hilfe dessen die lichtführende Faseranordnung 2 mit der Lichtaustrittsfläche - unter Mikroskopüberwachung durch den Operateur - im Auge positioniert werden kann.

Bei dem in den Figuren 1 und 2 dargestellten Ausführungsbeispiel ist auf das distale Ende der lichtleitenden Faseranordnung 2 eine Hülse 6 mit einer abwinkelbaren Andrücklippe 7 aufgesteckt, wobei die Andrücklippe 7 im Ruhezustand gegenüber der Achse der Faseranordnung schräg nach außen geneigt ist, sodass sie sich beim Andrücken an das Trabekelwerk 8 nur wie in Fig. 2 gezeigt nach außen abwinkeln kann. Durch diese Andrücklippe 7 kann nicht nur ein vorgegebener Anpressdruck eingestellt werden bzw. sich einstellen, sondern der Operateur erkennt durch das Abwinkeln dieser Andrücklippe auch das erfolgte Anliegen der Lichtaustrittsfläche 3 am Trabekelwerk 8 und kann dann die Laserquelle einschalten.

Bei der in Fig. 3 gezeigten Ausführungsform - die gegebenen Falls auch zusätzlich noch die Andrückvorrichtung mit der Andrücklippe 7 nach den Fig. 1 und 2 enthalten könnte - ist der Laserkatheder im Bereich des Handgriffs 5 zweigeteilt, wobei die Teile 5a und 5b axial gegen die Wirkung einer Feder 9 gegeneinander verschiebbar sind. Ein zu festes Andrücken der Spitze der lichtleitenden Faseranordnung 2 mit der Gefahr eines Durchstoßens durch das Gewebe, kann durch Einstellung der Federkraft der Feder 9 verhindert werden. Dabei kann gegebenen Falls auch eine nicht gezeigte Überlastsicherung vorgesehen sein, die erst bei Überschreiten eines vorgegebenen Anpressdrucks auslöst und dann die freie Verschiebbarkeit der Teile 5a und 5b gegeneinander freigibt.

Nicht gezeigt ist auch eine optische oder akustische Anzeigevorrichtung zur Signalisierung einer Überschreitung eines vorgegebenen Anpressdrucks, indem entweder eine Leuchtdiode aufleuchtet oder ein akustisches Signal ertönt, das den Operateur darauf aufmerksam macht, dass ein weiteres Vorwärtsbewegen des Laserkatheders zur Verhinderung einer Drucküberschreitung unterbleiben muss.

Eine Variante der Ausführung in Fig. 3 könnte eine Vorrichtung sein, die mit nichtfedernden Bauteilen (z. B. Membran-/ Kristalltechnik), welche ebenfalls drucksensitiv sind, realisiert ist.

Die Fig. 4 zeigt schließlich schematisch das distale Ende der lichtleitenden Faseranordnung 2 mit einem seitlich angeklebten vorne überstehenden Dorn 10, der durch das Einstechen in das Trabekelwerk 8 eine Positioniersicherung bewirkt, sodass ein seitliches Verrutschen der Lichtaustrittsfläche 3 und damit eine Veränderung der Position vor dem im Trabekelwerk liegenden Schlemm'schen Kanal verhindert wird.

Der Dorn kann selbstverständlich auch an einem hülsenförmigen Bauteil befestigt oder angeformt sein, das auf die Spitze der lichtleitenden Faseranordnung 2 aufsteckbar ist.

Die Erfindung ist nicht auf die dargestellten Ausführungsbeispiele beschränkt. So wäre es insbesondere auch möglich, ein Bauteil mit oder ohne abwinkelbare Andrücklippe wie beim Ausführungsbeispiel nach den Figuren 1 und 2 auf das distale Ende der lichtleitenden Faseranordnung 2 aufzustecken, die aus einem Antirutschmaterial besteht, das am Trabekelwerk haftet. Ein solches Material kann beispielsweise ein Material mit einer bestimmten Mikrostruktur sein, die ein solches Abrutschen verhindern kann.

## Patentansprüche

1. Vorrichtung zur Glaukombehandlung mit einem Laserkatheder und einer lichtführenden Faseranordnung, an deren proximalem Ende Licht einkoppelbar ist und an deren distalem Ende eine Lichtaustrittsfläche vorgesehen ist, **gekennzeichnet durch** eine drucksensitive Vorrichtung (6, 7, 9) zur Erzielung eines vorgegebenen Anlagedrucks der Lichtaustrittsfläche (3).

2. Vorrichtung nach Anspruch 1, **dadurch gekennzeichnet, dass** das distale Ende ein elastisches Bauteil (6) mit einer abwinkelbaren Andrücklippe (7) aufweist.

3. Vorrichtung nach Anspruch 2, **dadurch gekennzeichnet, dass** die Andrücklippe (7) so ausgestaltet ist, dass sie im Kammerwinkel abstützend wirkt und damit ein Abgleiten der Faseranordnung an der zuvor platzierten Stelle verhindert.

4. Vorrichtung nach einem der Ansprüche 1 bis 3, **dadurch gekennzeichnet, dass** der Laserkatheder (1) im Bereich außerhalb des Auges zwei axial gegen eine Federanordnung (9) gegeneinander verschiebbare Abschnitte (5a, 5b) aufweist.

5. Vorrichtung nach einem der Ansprüche 1 bis 3, **dadurch gekennzeichnet, dass** der Laserkatheder (1) im Bereich außerhalb des Auges eine drucksensitive Vorrichtung aufweist, die mit nichtfedernden Bauteilen (z. B. Membran-/ Kristalltechnik), welche ebenfalls drucksensitiv sind, realisiert ist.

6. Vorrichtung nach Anspruch 4, **gekennzeichnet durch** eine Überlastsicherung die bei Überschreiten eines vorgebbaren Anpressdrucks der Lichtaustrittsfläche (3) der lichtführenden Faseranordnung (2) die kraftschlüssige Verbindung der Abschnitte (5a, 5b) unterbricht.

7. Vorrichtung nach einem der Ansprüche 4 bis 6, **gekennzeichnet durch** eine optische und/oder akustische Anzeigevorrichtung zur Signalisierung einer Erreichung oder Überschreitung des vorgegebenen Anpressdrucks.

8. Vorrichtung insbesondere zur Glaukombehandlung mit einem Laserkatheder und einer lichtführenden Faseranordnung, an deren proximalem Ende Licht einkoppelbar ist und an deren distalem Ende eine Lichtaustrittsfläche vorgesehen ist, **dadurch gekennzeichnet, dass** das distale Ende der lichtführenden Faseranordnung mit einer ein Abgleiten der angedrückten Lichtaustrittsfläche verhindernden, vorzugsweise aufsteckbaren Abgleitsicherung versehen ist.

9. Vorrichtung nach Anspruch 8, **dadurch gekennzeichnet, dass** die Abgleitsicherung einen Dorn (10) aufweist.

10. Vorrichtung nach Anspruch 8, **dadurch gekennzeichnet, dass** die Abgleitsicherung aus einem Antirutschmaterial besteht, das am Trabekelwerk haftet.
